# FASCICULE DE BREVET EUROPEEN

(11) **EP 1 320 338 B1**
(45) Date de publication et mention de la délivrance du brevet: **17.05.2006**
(21) Numéro de dépôt: 01974401.0
(22) Date de dépôt: 27.09.2001
(51) Int. Cl.: A61F 2/16

(54) **INJECTEUR D'IMPLANT INTRAOCULAIRE SOUPLE**
EINFÜHRUNGSVORRICHTUNG ZUM IMPLANTIEREN EINER WEICHEN INTRAOKULARLINSE
FLEXIBLE INTRAOCULAR IMPLANT INJECTOR

(30) Priorité: 28.09.2000 FR 0012349
(43) Date de publication de la demande: 25.06.2003
(73) Titulaire: CORNEAL INDUSTRIE, 74370 Pringy (FR)
(72) Inventeur: JEANNIN, Lionel, F-74940 Ancy le Vieux (FR); VITALLY, Guy, F-74370 Villaz (FR)
(74) Mandataire: Dronne, Guy
(86) Numéro de dépôt international: PCT/FR2001/002994
(87) Numéro de publication internationale: WO 2002/026167

(56) Documents cités:
- WO-A-99/59668
- DE-A- 19 904 220

## Description

La présente invention a pour objet un injecteur d'implant intraoculaire souple et notamment un piston pour un tel injecteur.

Les implants intraoculaires sont des systèmes de correction de la vision constitués par une partie optique et par une partie haptique, cette dernière servant à maintenir dans l'oeil l'implant de telle manière que sa partie optique présente un axe qui coïncide avec l'axe de l'oeil du patient équipé de l'implant. L'implant est placé le plus souvent dans le sac capsulaire de l'oeil après ablation du cristallin mais il peut encore être mis en place dans la chambre antérieure ou dans la chambre postérieure de l'oeil du patient.

Afin de diminuer la dimension de l'incision nécessaire à la mise en place de l'implant à l'intérieur de l'oeil, des implants intraoculaires comportant une partie optique souple réalisée en matériaux acryliques hydrophiles ou hydrophobes ou encore en silicone, ont été développés. En effet, avec de tels implants, il est possible de plier ou de rouler la partie optique de l'implant avant son introduction dans l'oeil, ce qui permet bien sûr de diminuer l'encombrement total de l'implant lors de son introduction dans l'oeil.

En ce qui concerne la partie haptique de l'implant qui a, comme on l'a expliqué, une fonction uniquement mécanique, elle peut se présenter de différentes manières. Dans certains cas, la partie haptique est constituée par deux éléments haptiques également réalisés en matériau souple qui peuvent donc être roulés ou pliés simultanément avec la partie optique. Dans d'autres cas, la partie haptique peut être constituée par deux branches haptiques présentant une courbure régulière et réalisées en un matériau intrinsèquement rigide tel que du PMMA et dont les propriétés de flexibilité sont dues à la faible dimension transversale de ces branches.

Pour aider le chirurgien lors de la mise en place de l'implant dans l'oeil, on a développé des dispositifs appelés injecteurs d'implants intraoculaires qui remplissent la double fonction de réaliser l'enroulement ou le pliage de l'implant et l'introduction de l'implant dans l'oeil après son pliage par l'intermédiaire d'une canule introduite dans l'incision réalisée dans la cornée de l'oeil.

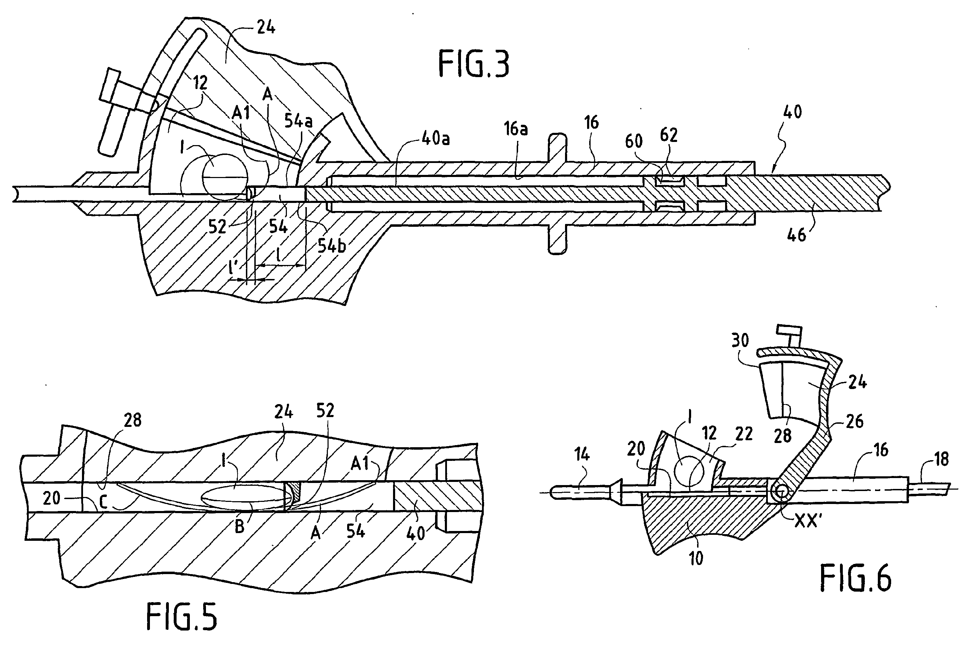

La figure 6 montre un exemple de réalisation d'un tel injecteur d'implant. II comprend un corps 10 qui définit une chambre de pliage 12 prolongée à l'une de ses extrémités par une canule 14 et à son autre extrémité par un canal de guidage 16 pour un piston mobile 18. Dans ce mode de réalisation, la chambre de pliage 12 comporte essentiellement une paroi de pliage 20 de forme hémi-cyclindrique et une surface plane 22 de réception de l'implant. L'injecteur comporte également un poussoir mobile de pliage 24 qui, dans ce cas particulier, est monté pivotant autour d'un axe X-X' par l'intermédiaire d'un bras 26. Le poussoir 24 comporte une face active hémi-cylindrique 28 et une surface plane 30 parallèle à la surface plane 22 de la chambre d'enroulement. On comprend que lorsque l'implant I est disposé dans la chambre d'enroulement sur la surface plane 22, en faisant pivoter le poussoir 24 autour de son axe X-X', la face active hémi-cylindrique de ce poussoir coopère avec un des bords de l'implant I qui est pris entre les deux surfaces hémi-cylindriques 20 et 28. Le déplacement du poussoir provoque l'enroulement de l'implant jusqu'à ce que celui-ci soit totalement enroulé ou plié dans le volume cylindrique limité par les deux surfaces hémi-cylindriques 20 et 28. Lorsque l'enroulement ou le pliage de l'implant a été réalisé, celui-ci peut être poussé à l'aide du piston 18 dans la canule 14 en vue de son introduction dans l'oeil du patient.

Lorsque les éléments haptiques sont relativement massifs et, par exemple, réalisés en matériaux souples et donc pliés en même temps que la partie optique, l'élément haptique au contact de l'extrémité du piston 18 présente une résistance mécanique suffisante pour assurer que l'action du piston sur cet élément haptique n'entraînera aucune altération de l'élément haptique lors du déplacement de l'implant plié à l'aide du piston. En revanche, dans le cas où la partie haptique est constituée par deux branches courbées en matériau rigide et présentant une section transversale réduite, l'action de l'extrémité du piston 18 sur l'extrémité de la branche haptique risque d'endommager cette branche haptique et donc de rendre l'implant lui-même inutilisable.

Le document WO-A-99/59668 décrit un injecteur d'implants souples. L'extrémité du piston de cet injecteur comporte des évidements pour recevoir une anse de l'implant souple, mais ces évidements ne permettent pas d'obtenir une protection mécanique de l'anse de l'implant.

Un objet de la présente invention est de fournir un dispositif d'injection d'implant intraoculaire et plus particulièrement un piston pour un tel injecteur, qui soit adapté aux cas d'implants intraoculaires comportant des branches haptiques de résistance mécanique limitée et qui puisse être utilisé dans un injecteur d'implants du type décrit en liaison avec la figure 6 ou d'un autre type dans lequel l'implant est plié ou enroulé dans l'injecteur avant l'action du piston permettant de pousser l'implant plié.

Pour atteindre ce but selon l'invention, le dispositif d'injection d'un implant intraoculaire dans l'oeil d'un patient qui comprend une partie optique souple et deux branches haptiques ayant une première extrémité raccordée à ladite partie optique et une deuxième extrémité libre, comprend une chambre de pliage de la partie optique de l'implant, un organe de pliage, une canule d'injection de l'implant plié dans l'oeil, ladite canule débouchant à une première extrémité de ladite chambre, un canal de guidage débouchant à la deuxième extrémité de ladite chambre, et un piston mobile monté dans ledit canal de guidage pour pousser ledit implant plié dans ladite canule, ledit piston comprenant une extrémité cylindrique pour coopérer avec ledit implant, ladite extrémité comportant une face d'extrémité, sensiblement orthogonale à la longueur du piston, ladite face d'extrémité présentant une ouverture débouchant dans la paroi latérale de l'extrémité du piston et s'étendant sur une partie seulement du diamètre du piston. Le dispositif se caractérise en ce que ladite extrémité étant munie d'une fente s'étendant sur une longueur l selon la direction de l'axe du piston, ladite longueur I étant telle que, avec la longueur de ladite ouverture, la longueur totale soit au moins égale à celle de la branche haptique, ladite fente communiquant avec l'ouverture et s'étendant en outre sur tout le diamètre du piston pour déboucher à ses extrémités diamétrales dans la paroi externe de l'extrémité dudit piston, ladite fente et ladite ouverture ayant la même largeur et le même plan médian diamétral.

On comprend que grâce à la présence de l'ouverture dans la face d'extrémité du piston et de la fente dans l'extrémité de celui-ci, la branche haptique de l'implant intraoculaire peut être mise en place à travers cette ouverture et cette fente, avant le pliage de l'implant intraocutaire. Lors de l'opération de pliage, l'optique souple est enroulé ou plié et la branche haptique est élastiquement déformée pour prendre une forme proche d'un segment de droite en étant logée dans l'ouverture et dans la fente du piston. Ainsi, lorsque l'on veut procéder à l'injection de l'implant dans l'oeil du patient, la face d'extrémité du piston, agira directement sur la périphérie de la partie optique pliée sans introduire de contrainte mécanique dans l'anse haptique.

D'autres caractéristiques et avantages de l'invention apparaîtront mieux à la lecture de la description qui suit d'un mode de réalisation de l'invention donné à titre d'exemple non limitatif. La description se réfère aux figures annexées sur lesquelles :
la figure 1 est une vue en perspective d'un piston pour injecteur d'implant selon l'invention ;
la figure 2 est une vue en perspective de l'extrémité de la tige du piston ;
la figure 3 est une vue en coupe longitudinale de l'ensemble de l'injecteur d'implant équipé du piston représenté sur la figure 1 ;
la figure 4 est une vue de détail montrant l'engagement de la branche haptique de l'implant intraoculaire dans l'ouverture et la fente de l'extrémité du piston ;
la figure 5 est une coupe longitudinale partielle de l'injecteur d'implant montrant celui-ci après la phase d'enroulement ou de pliage ; et
la figure 6, déjà décrite, montre un exemple de réalisation d'un injecteur d'implant intraoculaire.

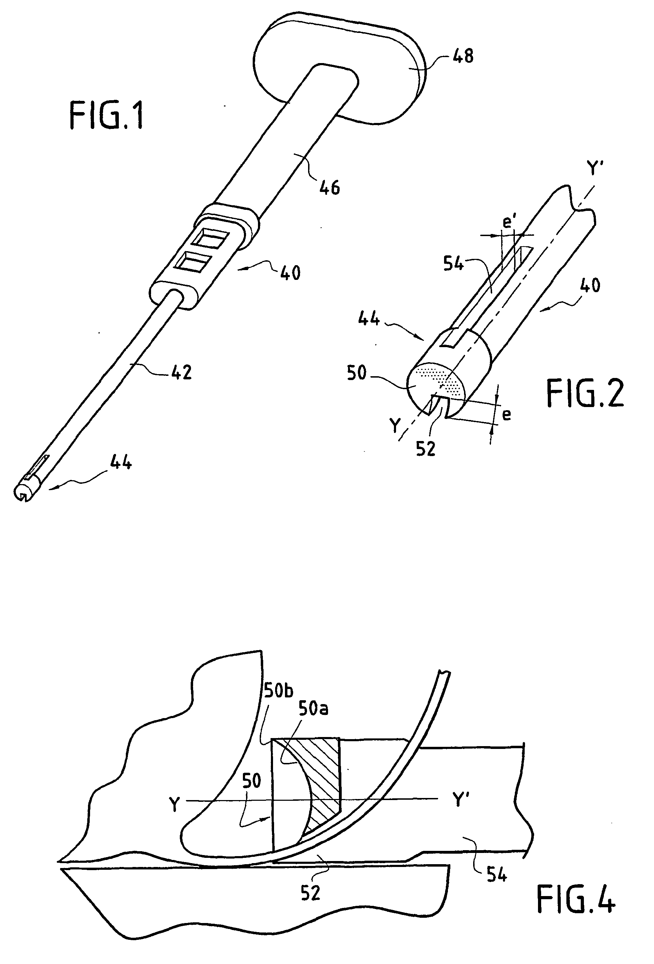

Sur la figure 1, on a représenté un piston 40 pour injecteur d'implant. Ce piston comporte une tige cylindrique 42 se terminant par une extrémité 44. Le piston comporte également un corps plus massif 46 terminé par une surface 48 permettant de pousser le piston à l'intérieur du corps de l'injecteur.

Sur la figure 2, on a représenté l'extrémité 44 du piston 40. Celle-ci comporte une face d'extrémité 50 qui est sensiblement disposée dans un plan orthogonal à l'axe Y-Y' du piston. La face d'extrémité 50 du piston est munie d'une ouverture 52 et l'extrémité du piston est munie d'une fente diamétrale 54.

Sur la figure 3, on a représenté l'ensemble d'un injecteur conforme à celui qui a été décrit en liaison avec la figure 6 mais muni d'un piston 40 conforme à celui qui est représenté sur les figures 1 et 2. L'injecteur est du type représenté sur la figure 6. II va cependant de soi que cet injecteur pourrait être d'un autre type, dès lors que l'implant se trouve plié ou enroulé dans l'injecteur et que le piston agit sur un bord de l'implant plié. Sur cette figure, on voit que l'ouverture 52 est disposée selon un plan diamétral du piston et s'étend sur une longueur e selon la direction diamétrale inférieure au diamètre du piston et, de préférence, au maximum de l'ordre du rayon de celui-ci. Dans un mode particulier de réalisation, le piston a un diamètre égal à 2 mm, l'ouverture 52, dans la face d'extrémité, a une longueur e selon la direction diamétrale de 0,5 mm. La fente 54 communique avec l'ouverture 52 et s'étend sur toute la longueur du diamètre du piston. Il en résulte que les deux extrémités diamétrales 54a et 54b de cette fente débouchent dans la paroi externe 40a du piston. L'ouverture 52 présente une longueur axiale l' et la fente 54 présente une longueur axiale I. Ces longueurs l' et I sont déterminées de telle manière que la somme de leur longueur soit au moins égale à la longueur de la branche haptique A de l'implant intraoculaire I. La somme de ces longueurs peut être égale à 10 mm.

De préférence, l'ouverture 52 et la fente 54 ont une même largeur e' en section par des plans orthogonaux à l'axe YY' du piston. Cette largeur est bien sûr supérieure à l'épaisseur de la branche haptique de l'implant. Cette largeur peut être de l'ordre de 0,3 mm.

Comme le montre mieux la figure 4, la face d'extrémité 50 du piston, de préférence, n'est pas plane mais constituée par une calotte sphérique 50a d'axe Y-Y', cette calotte sphérique étant concave.

Cette figure montre également que, de préférence, la dimension diamétrale e de l'ouverture 52 va en augmentant de la face d'extrémité 50 vers la fente 54. Cette disposition facilite l'introduction de la branche de l'implant dans l'ouverture 52.

Grâce au fait que l'ouverture 52 ne correspond qu'à une fraction du diamètre du piston, la branche de l'implant reste bien maintenue dans l'ouverture 52 et la fente 54. En outre, c'est bien la périphérie de la face d'extrémité 50 qui est en appui sur la périphérie de la partie optique de l'implant.

Comme le montre mieux la figure 3, de préférence le corps 46 du piston est muni de moyens de solidarisation temporaire, de préférence des moyens de clipsage temporaire 60 qui peuvent coopérer avec des moyens de solidarisation temporaire, de préférence de clipsage temporaire 62 prévus dans la face interne 16a du canal de guidage 16 de l'injecteur d'implant. Ce système de clipsage temporaire permet d'immobiliser temporairement en translation l'ensemble du piston 40 dans une position telle que l'intégralité de la fente 54 de l'extrémité du piston soit disposée à l'intérieur de la chambre d'enroulement 12 de l'injecteur d'implant intraoculaire. C'est dans cette position que l'on a représenté le piston 40 sur la figure 3. Dans cette position, aucune contrainte mécanique n'est appliquée à l'implant ou à sa partie haptique. Dans cette position du piston, l'implant peut donc être stocké à l'intérieur de l'injecteur.

On va maintenant décrire l'utilisation de l'injecteur représenté sur les figures 2 à 5. Le piston 40 étant dans la position représentée sur la figure 3 et maintenu dans cette position grâce au système de clipsage temporaire 60, 62, le poussoir 24 de l'injecteur est complètement écarté comme cela est représenté sur la figure 6. On met alors en place l'implant I sur la surface plane 22 de la chambre d'enroulement en engageant l'extrémité libre A1 de la branche haptique A, d'abord dans l'ouverture 52, puis dans la fente 54. Après cette opération, l'implant occupe la position représentée sur la figure 3 et l'extrémité libre A1 de l'anse haptique A fait saillie hors de la fente 54.

Sur la figure 5, on a représenté un injecteur d'implant lorsque le poussoir 24 se trouve dans sa position finale dans laquelle les surfaces hémi-cylindriques 20 et 28 définissent la chambre d'enroulement proprement dite, c'est-à-dire que la partie optique B de l'implant I est enroulée ou pliée et l'anse haptique A2, préalablement introduite dans la fente 54, subit une contrainte qui provoque sa déformation pour l'amener dans une position sensiblement rectiligne à l'intérieur de la fente 54 et de l'ouverture 52 et la deuxième anse haptique C a subi exactement la même déformation.

On comprend que pour réaliser l'introduction de l'implant plié dans l'oeil du patient, il suffit d'introduire la canule 14 dans l'incision pratiquée dans la cornée du patient. Puis le chirurgien enfonce le piston 16 ce qui a pour effet que la face d'extrémité 50 du piston appuie sur le bord de la partie optique enroulée. Ainsi le piston n'exerce aucune contrainte mécanique sur l'anse haptique A. Cette poussée permet le déplacement de l'implant dans la canule et son introduction dans l'oeil où l'implant intraoculaire reprend sa forme initiale.

Il faut également souligner que, de préférence, la face d'extrémité 50 du piston a la forme d'une calotte sphérique concave. La périphérie 50b de cette calotte sphérique fait donc un angle obtus avec la paroi interne du canal de guidage et de la chambre d'enroulement. Cela évite les risques de pincement entre l'extrémité du piston et la paroi interne du canal ou de la chambre d'enroulement lors de l'enfoncement du piston.

## Revendications

1. Dispositif d'injection d'implant intraoculaire dans l'oeil d'un patient, l'implant comprenant une partie optique souple et deux branches haptiques ayant une première extrémité raccordée à ladite partie optique et une deuxième extrémité libre, ledit dispositif comprenant une chambre de pliage (12) de la partie optique de l'implant, un organe de pliage (20), une canule d'injection (14) de l'implant plié dans l'oeil, ladite canule débouchant à une première extrémité de ladite chambre, un canal de guidage (16) débouchant à la deuxième extrémité de ladite chambre, et un piston mobile (40) monté dans ledit canal de guidage pour pousser ledit implant plié dans ladite canule, ledit piston comprenant une extrémité cylindrique (44) pour coopérer avec ledit implant, ladite extrémité comportant une face d'extrémité (50), sensiblement orthogonale à la longueur du piston, ladite face d'extrémité présentant une ouverture (52) débouchant dans la paroi latérale de l'extrémité du piston et s'étendant sur une partie seulement du diamètre du piston, ledit dispositif étant **caractérisé en ce que** ladite extrémité (44) du piston est munie d'une fente (54) s'étendant sur une longueur selon la direction de l'axe du piston, ladite longueur 1 étant telle que, avec la longueur de ladite ouverture, la longueur totale soit au moins égale à celle de la branche haptique, ladite fente communiquant avec ladite ouverture et s'étendant en outre sur tout le diamètre du piston pour déboucher à ses extrémités diamétrales (54a, 54b) dans la paroi externe (40a) de l'extrémité dudit piston, ladite fente (54) et ladite ouverture (52) ayant la même largeur et le même plan médian diamétral.

2. Dispositif selon la revendication 1, **caractérisé en ce que** ladite face d'extrémité (50) a la forme d'une calotte sphérique concave ayant pour axe l'axe dudit piston (40).

3. Dispositif selon l'une quelconque des revendications 1 et 2, **caractérisé en ce que** ledit piston (40) et la paroi interne dudit canal de guidage comportent des moyens de solidarisation temporaire (62) pour maintenir ledit piston dans une position telle que la totalité de la fente (54) du piston soit dans ladite chambre de pliage.

4. Dispositif selon l'une quelconque des revendications 1 à 3, **caractérisé en ce que** la dimension (e) de ladite ouverture (52) selon la direction diamétrale du piston, dans ladite face d'extrémité (50) est au plus égale au rayon du piston.

5. Dispositif selon l'une quelconque des revendications 1 à 4, **caractérisé en ce que** la dimension de ladite ouverture (52) selon la direction diamétrale du piston (40) va en augmentant de ladite face d'extrémité (50) à ladite fente (54).

## Claims

1. An injector device for injecting an intraocular implant into the eye of a patient, the implant comprising a flexible optical portion and two haptic branches each having a first end connected to said optical portion and a second end that is free, said device comprising a folding chamber (12) for folding the optical portion of the implant, a folding member (20), a hollow needle (14) for injecting the folded implant into the eye, said hollow needle opening out at a first end of said chamber, a guide channel (16) opening out at the second end of said chamber, and a moving piston (40) mounted in said guide channel so as to push said folded implant into said hollow needle, said piston including a cylindrical end (44) for cooperating with said implant, said end including an end face (50) that is substantially orthogonal to the length of the piston, said end face presenting an opening (52) opening out in the side wall of the end of the piston and extending over a portion only of the diameter of the piston, said device being **characterised in that** said end (44) of the piston is provided with a slot (54) extending over a length in the axial direction of the piston, said length ℓ being such that, together with the length of said opening, the total length is not less than the length of the haptic branch, said slot communicating with said opening and also extending over the entire diameter of the piston so as to open out at its diametral ends (54a, 54b) in the outside wall (40a) of the end of said piston, said slot (54) and said opening (52) having the same width and the same diametral median plane.

2. A device according to claim 1, **characterised in that** said end face (50) is in the shape of a concave spherical cap, having the same axis as said piston (40).

3. A device according to claim 1 or claim 2, **characterised in that** said piston (40) and the inside wall of said guide channel include temporary retention means (62) for holding said piston in a position such that the entire slot (54) of the piston is in said folding chamber.

4. A device according to any one of claims 1 to 3, **characterised in that** the dimension (e) of said opening (52) in the diametral direction of the piston, in said end face (50), is not greater than the radius of the piston.

5. A device according to any one of claims 1 to 4, **characterised in that** the dimension of said opening (52) in the diametral direction of the piston (40) increases progressively from said end face (50) to said slot (54).

## Patentansprüche

1. Vorrichtung zur Injektion eines Intraokularimplantats in das Auge eines Patienten, umfassend einen weichen optischen Teil und zwei haptische Schenkel, die ein erstes Ende, das an den optischen Teil angeschlossen ist, und ein zweites freies Ende aufweisen, wobei die Vorrichtung eine Kammer (12) zum Falten des optischen Teils des Implantats, ein Faltelement (20), eine Kanüle (14) zur Injektion des gefalteten Implantats in das Auge, wobei die Kanüle an einem ersten Ende der Kammer endet, einen Führungskanal (16), der am zweiten Ende der Kammer mündet, und einen beweglichen Kolben (40) umfaßt, der in dem Führungskanal befestigt ist, um das gefaltete Implantat in die Kanüle zu schieben, wobei der Kolben ein zylindrisches Ende (44) umfaßt, um mit dem Implantat zusammenzuwirken, wobei das Ende eine im wesentlichen zur Länge des Kolbens orthogonale Endfläche (50) umfaßt, wobei die Endfläche eine Öffnung (52) aufweist, die in die Seitenwand des Endes des Kolbens mündet und sich nur über einen Teil des Durchmessers des Kolbens erstreckt, wobei die Vorrichtung **dadurch gekennzeichnet ist, daß** das Ende (44) des Kolbens mit einem Schlitz (54) versehen ist, der sich auf einer Länge in Richtung der Achse des Kolbens erstreckt, wobei die Länge 1 derart ist, daß mit der Länge der Öffnung die Gesamtlänge mindestens gleich jener des haptischen Schenkels ist, wobei der Schlitz mit der Öffnung in Verbindung steht und sich ferner über den gesamten Durchmesser des Kolbens erstreckt, um an seinen diametralen Enden (54a, 54b) in die Außenwand (40a) des Endes des Kolbens zu münden, wobei der Schlitz (54) und die Öffnung (52) dieselbe Breite und dieselbe diametrale Mittelebene aufweisen.

2. Vorrichtung nach Anspruch 1, **dadurch gekennzeichnet, daß** die Endfläche (50) die Form einer konkaven Kugelkappe hat, die als Achse die Achse des Kolbens (40) hat.

3. Vorrichtung nach einem der Ansprüche 1 und 2, **dadurch gekennzeichnet, daß** der Kolben (40) und die Innenwand des Führungskanals zeitweilige Befestigungsmittel (62) umfassen, um den Kolben in einer derartigen Position zu halten, daß die Gesamtheit des Schlitzes (54) des Kolbens in der Faltkammer liegt.

4. Vorrichtung nach einem der Ansprüche 1 bis 3, **dadurch gekennzeichnet, daß** die Abmessung (e) der Öffnung (52) in diametraler Richtung des Kolbens in der Endfläche (50) höchstens gleich dem Radius des Kolbens ist.

5. Vorrichtung nach einem der Ansprüche 1 bis 4, **dadurch gekennzeichnet, daß** die Abmessung der Öffnung (52) in diametraler Richtung des Kolbens (40) von der Endfläche (50) zum Schlitz (54) größer wird.
